## Europäisches Patentamt
## European Patent Office
## Office européen des brevets

(19)

(11) Publication number: **0 030 088**
**B1**

# EUROPEAN PATENT SPECIFICATION

(12)

(45) Date of publication of patent specification: **30.06.82**

(21) Application number: **80304000.5**

(22) Date of filing: **10.11.80**

(51) Int. Cl.³: **C 07 C 49/04,**
**C 07 C 45/53,**
**C 07 C 39/04,**
**C 07 C 37/08,**
**C 07 C 15/107, C 07 C 2/66**
**//C07C178/00, C07C179/035**

(54) Co-production of 2-alkanones and phenols.

(30) Priority: **30.11.79 US 99344**

(43) Date of publication of application:
**10.06.81 Bulletin 81/23**

(45) Publication of the grant of the patent:
**30.06.82 Bulletin 82/26**

(84) Designated Contracting States:
**BE DE FR GB IT NL**

(56) References cited:
**GB - A - 1 132 859**
**GB - A - 1 496 227**
**US - A - 4 197 413**

(73) Proprietor: **MOBIL OIL CORPORATION**
**150 East 42nd Street**
**New York New York 10017 (US)**

(72) Inventor: **Haag, Werner Otto**
**38 Pine Knoll Drive**
**Lawrenceville New Jersey 08048 (US)**
Inventor: **Young, Lewis Brewster**
**Pineview Court**
**Skillman New Jersey 08558 (US)**

(74) Representative: **Cooper, John Anthony**
**Mobil Court 3 Clements Inn**
**London WC2A 2EB (GB)**

Courier Press, Leamington Spa, England.

## Co-production of 2-alkanones and phenols

This invention is directed to a process for the production of oxygenated organic compounds. In particular, it is concerned with the selective production of 2-alkanones and phenols by a process utilizing crystalline zeolite catalysts.

The relatively long chain 2-alkanones, i.e. those having five or more carbon atoms in the primary chain, are generally prepared by complex multistep processes or in a reaction which leads to a mixture of products. For example, the reaction of acetaldehyde with ethylene leads to a mixture of products including 2-butanone, 2-hexanone and other higher 2-alkanones with even numbers of carbon atoms.

Phenols are traditionally prepared by the oxidation of alkylbenzenes (e.g. isopropylbenzene) and the acid catalyzed rearrangement of the resultant hydroperoxide to the corresponding phenol and ketone. For example, isopropylbenzene (cumene) can be oxidized to form phenol and acetone through the intermediate cumene hydroperoxide.

2-Alkanones are useful in agriculture as herbicides. For instance, 2-dodecanone is known to be an effective cotton defoliant. Other 2-alkanones are useful as chemical intermediates. Phenol is a large volume chemical product which is used to manufacture various resins. Methyl substituted phenols are also produced commercially in relatively large volume.

We have now discovered a process for coproducing relatively long chain length 2-alkanones and phenols or substituted phenols. In the first step of the process, benzene or a substituted benzene is aklylated with an alkylating agent (e.g. an olefin) having at least five carbon atoms. The alkylation reaction is carried out in the presence of a member of a shape selective crystalline zeolite catalyst which affects the reaction so that the resultant alkylbenzene product is rich in the 2-arylakane isomer.

The crystalline zeolite catalysts which are used in the present process have crystal structures with channels or networks of pores with openings having a major dimension of from 6 to 7 Angstrom units. The zeolites may be naturally occurring or synthetic in origin. Specific preferred members of this class are cancrinite, gmelinite, mordenite, dealuminized mordenite and offretite, as well as their synthetic and naturally occurring isotypes such as zeolite S, zeolite Na-S, zeolite Na-D, Ptilolite, Zeolon, zeolite O and TMA-offretite. A particularly preferred zeolite is the synthetic zeolite ZSM—12.

In the second step of the process, the alkylation product of the first step is oxidized to a hydroperoxide. The hydroperoxide is thereafter subjected to acid-catalyzed rearrangement (the third step) to yield the desired 2-alkanone having the same number of carbon atoms as the original alkylating agent, together with the phenolic compound. Both the second and third steps utilize conventional technology such as is used, for example, in the coproduction of acetone and phenol from isopropylbenzene.

The alkylating agents useful in the present process include aliphatic or aromatic organic compounds, having one or more available alkyl groups of at least five carbon atoms and which are capable of reacting with an aromatic compound. Useful alkylating agents include, for example, alkyl halides, olefins or alcohols having a linear hydrocarbon chain length or "backbone" of at least five (5) carbon atoms, and preferably from 6 to 20 carbon atoms. Olefins are the preferred alkylating agents, although other hydrocarbon material which will generate unsaturated carbon atoms in the presence of the alkylation catalysts may also be used.

The aromatic compounds which are to be reacted with the alkylating agent to yield the 2-phenylalkanes are generally benzene compounds. These benzene compounds may be unsubstituted, or they may carry from 1 to 2 substituents on the aromatic ring. If substituted, the substituent may be, for example, an alkyl group having from 1 to 10 carbon atoms, a halide, an alkoxy, or an aryl group or any combination of such substituents.

The crystal structure of the zeolites suitable for use as catalysts in the present process provides access to and egress from the intracrystalline free space of the zeolites through channels or networks of pores (hereinafter referred to as pores). The openings of such pores have a major dimension from 6A and 7A (0.6 to 0.7 nm.). These zeolites have pore apertures of the size provided by 12-member rings of silicon or aluminum atoms. These rings are formed by the regular disposition of the tetrahedra making up the anionic framework of the crystalline zeolite, the silicon or aluminum atoms forming the centers of the tetrahedra and being themselves bonded together by oxygen atoms.

The pores in the zeolites may be substantially circular, such as those in cancrinite which has substantially uniform pores of about 6.2 Angstroms in diameter, or they may be somewhat elliptical, such as in mordenite which has pores of approximately 6.7 by 7.0 Angstroms. The zeolites used in the present process have, however, a major pore dimension intermediate that of the large pore zeolites, such as the X and Y zeolites, and the relatively small pore size zeolites ZSM—5 and ZSM—11. With the exception of zeolite ZSM—12, the pore size dimensions and crystal structures of the above zeolites are substantially those specified in ATLAS OF ZEOLITE STRUCTURE TYPES by W. M. Meier and D. H. Olson, published by the Structure Commission of the International Zeolite Association (1978) and distributed by Polycrystal Book Service, Pittsburgh, Pennsylvania. The structure and pore size of ZSM—12 have not been finally established but on the basis of present information is believed to be within the above limits. ZSM—12 is described in U.S. Patent No. 3,832,449.

The zeolites used in the present process generally have at least 10 percent of their cationic sites occupied by ions other than alkali or alkaline-earth metals. Typical but nonlimiting replacing ions include ammonium, hydrogen, rare earth, zinc, copper and aluminum. Of this group, particular preference is accorded ammonium, hydrogen, rare earth or combinations thereof. In a preferred embodiment, the zeolites are converted to the predominantly hydrogen form, generally by replacement of the alkali metal or other ion originally present with hydrogen ion precursors, e.g. ammonium ions, which upon calcination yield the hydrogen form. This exchange is conveniently carried out by contact of the zeolite with an ammonium, salt solution, e.g. ammonium chloride, utilizing well known ion exchange techniques. The extent of replacement is preferably such as to produce a zeolite material in which at least 50 percent of the cationic sites are occupied by hydrogen ions.

The zeolites may be subjected to various chemical treatments, including alumina extraction and combination with one or more metal components, particularly the metals of Groups IIB, III, IV, VI, VII and VIII. The zeolites may, in some instances, be subjected to thermal treatment, including steaming or calcination in air, hydrogen or an inert gas, e.g. nitrogen or helium.

An especially useful modifying treatment entails steaming of the zeolite by contact with an atmosphere containing from 5 to 100 percent steam at a temperature of from 250° to 1000°C. Steaming may last for a period of between 0.25 and 100 hours and may be conducted at pressures ranging from subatmospheric to several hundred atmospheres to reduce the alpha value of the zeolite to less than 500, and preferably less than 20, but greater than zero.

In practising the conversion process, it may be useful to incorporate the zeolite in another material resistant to the temperature and other conditions employed in the process. Such matrix materials include synthetic or naturally occurring substances as well as inorganic materials such as clay, silica, and/or metal oxides. The latter may be either naturally occurring or in the form of gels or gelatinous precipitates, including mixtures of silica and metal oxides. Naturally occurring clays which can be composited with the zeolite include those of the montmorillonite and kaoline families, including the subbentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment, or chemical modification.

The zeolites may also be compounded with a porous matrix material, such as alumina, silica-alumina, silica-magnesia, silica-zirconia, silica-thoria, silica-beryllia, and silica-titania, as well as ternary combinations, such as silica-alumina-thoria, silica-alumina-zirconia, silica-alumina-magnesia and silica-magnesia-zirconia. The matrix may be in the form of a cogel. The relative proportions of finely divided zeolite and inorganic oxide gel matrix may vary widely, with the zeolite content ranging from between 1 to 99 percent by weight and more usually in the range of 5 to 80 percent by weight of the composite.

The process of this invention is basically a three-step reaction. In Step 1 the aryl compound and alkylating agent are reacted in the presence of the shape selective zeolite to yield the product with a predominance of the 2-arylakane isomer. Step 2 is an oxidation step in which the 2-arylakane is oxidized with a suitable oxidizing agent to produce the corresponding hydroperoxide. In Step 3 the hydroperoxide is cleaved and rearranged in the presence of of an acid catalyst to yield the 2-alkanone of the same carbon chain length as the cleaved alkyl group and also a phenol based on the structure of the aryl moiety.

In the first (alkylation) step of the process the organic reactants, i.e. the aromatic compound and the alkylating agent, are brought into contact with the zeolite in a suitable reaction zone, such as a fixed bed of the catalyst, under effective alkylation conditions. Such conditions include a temperature of 50°C to 500°C, a pressure of from 25 to 25000 kPa and, when flow systems are contemplated, a feed weight hourly space velocity (WHSV) of from 0.1 to 500. The latter WHSV is based upon the weight of the catalyst compositions employed, i.e. the total weight of active catalyst and binder therefor. Preferred reaction conditions include a temperature within the approximate range of 100°C to 350°C with a feed WHSV of between 0.5 and 100. Although the reaction normally takes place at atmospheric pressure 100 kPa, the preferred pressure range extends from 100 to 5000 kPa. The reactants may be in either the vapor phase or the liquid phase and may be free from intentional admixture or dilution with other material, or they may be brought into contact with the zeolite with the aid of carrier gases or diluents such as, for example, hydrogen or nitrogen.

The alkylation step may be carried out as a batch-type, semi-continuous or continuous operation utilizing a fixed or moving bed catalyst system. A preferred embodiment entails use of a catalyst zone in which the hydrocarbon charge is passed concurrently or countercurrently through a moving bed of particle-form catalyst. The catalyst, after use, is preferably conducted to a regeneration zone where coke is burned from the used catalyst in an oxygen-containing atmosphere (such as air) at elevated temperature, after which the regenerated catalyst is recycled to the conversion zone for further contact with the organic reactants.

The following Examples are provided to illustrate Step 1 of the process of this invention.

### Example 1
(ZSM—12)

Benzene was alkylated with octene-1 in the presence of zeolite HZSM—12 (silica/alumina ratio = 90; 65 wt.% on alumina binder). The reaction was carried out in a flow reactor at 205°C and 1480 kPa. The reactants, at a benzene/octene mole ratio of 4:1, were passed across the catalyst at a feed WHSV of 30 $hr^{-1}$. Analysis of the effluent indicated that, at 99% octene-1 conversion, selectivity to phenyloctane was 53%. Composition of the phenyloctanes was: 92% 2-phenyloctane, 7% 3-phenyloctane, and 1% 4-phenyloctane, with 69% being linear phenyloctanes.

### Example 2
(AlCl₃)

Using conventional Friedel-Crafts technology, benzene and octene-1 (mole ratio 8:1) were reacted with $AlCl_3$ catalyst at 30°C and atmospheric pressure. Octene-1 conversion was 97% and selectivity to phenyloctane 73%. Isomeric composition of the phenyloctane was: 49% 2-phenyloctane, 28% 3-phenyloctane, and 23% 4-phenyloctane, with 100% being linear phenyloctanes.

### Example 3
(Mordenite, dealuminized)

A sample of mordenite (Norton Zeolon Type 100 H, silica/alumina mole ratio = 10) was air calcined for one hour at 400°C followed by one hour at 600°C. The material was refluxed for 20 hours with 0.5N HCl (50 ml of solution per gram of zeolite) and then refluxed for 20 hours with distilled water. The silica to alumina ratio of the resulting dealuminized mordenite was 93.

Benzene and octene-1 (mole ratio = 4:1) were passed over a sample of the above material at a feed WHSV of 30 $hr^{-1}$, 198°C and 1480 kPa. Conversion of the $C_8$ = was 100% with 76% selectivity to phenyloctanes. Isomeric composition of the phenyloctanes was: 71.7% 2-phenyloctane, 28.3% 3-phenyloctanes, and no detectable amount of 4-phenyloctane; 87% of the phenyloctane product was linear phenyloctanes.

### Example 4
(Mordenite, dealuminized)

Repeat of Example 3, except at a temperature of 155°C, pressure of 1550 kPa and WHSV = 90 $hr^{-1}$. Octene conversion was 99.3% and selectivity to phenyloctane 77%. Isomeric phenyloctane composition was: 86.6% 2-phenyloctane, 13.4% 3-phenyloctane, and no detectable amount of 4-phenyloctane; 96% of the phenyloctanes were linear.

### Example 5
(ZSM—12, steamed)

A sample of the same HZSM—12 used in Example 1 was steamed prior to use by passing steam over the catalyst at a pressure of one atmosphere (absolute) at 538°C for about seven hours. A benzene/octene-1 feed stream (mole ratio = 8:1) was passed over the steamed catalyst at 194°C, 4000 kPa and WHSV of 30 $hr^{-1}$. Conversion of octene was 88% with 83% selectivity to phenyloctane. The phenyloctane composition was as follows: 93% 2-phenyloctane, 6% 3-phenyloctane, and 1% 4-phenyloctane; 81% linear phenyl-substituted octanes.

### Example 6
(ZSM—11)

A sample of synthetic zeolite HZSM—11 (U.S. Patent No. 3,709,979), which has a major pore dimension of 5.5A, was placed in a flow reactor at 256°C. A feed stream of benzene and octene-1 (mole ratio = 4:1) was passed over the catalyst at 4345 kPa and a WHSV of 30 $hr^{-1}$. Conversion of octene-1 was 100%, but selectivity to phenyloctane was only 6%. Due to the low yield and the large number of products found, the isomeric phenyloctanes could not be positively identified.

As will be seen from the foregoing, the zeolite catalysts used in the present process - i.e. ZSM—12 and mordenite - selectively produce 2-phenyloctane in preference to the 3- and 4-isomers, as compared to the conventional $AlCl_3$ catalyst. Conversion rates were high and the yield of the linear product excellent. Zeolite HZSM—11, which has a pore opening of less than the desired 6 to 7 Angstroms, has poor selectivity to phenyloctanes in general.

### Examples 7—14

In a series of runs utilizing various zeolite materials, benzene was alkylated with dodecene-1. The feed stream was a 4:1 mole ratio mixture of benzene and dodecene-1 which was passed across each of the catalysts at WHSV of 30 $hr^{-1}$. The reaction temperatures and pressures are shown in Table I below, as are the level of $C_{12}$ = conversion and the selectivity to phenyl- dodecane. Table II summarizes the isomeric distribution of the phenyldodecane produced.

## TABLE I

### Catalyst Comparison — Benzene + Dodecene-1

| Example | Zeolite | Major Pore Dimension | Temp., °C | Pressure, kPa | $C_{12}$ = Conversion | Selectivity to $\phi$-$C_{12}$ |
|---------|---------|----------------------|-----------|---------------|-----------------------|--------------------------------|
| 7 | HZSM—12 | * | 200 | 1415 | 54% | 63% |
| 8 | Mordenite** | 7.0A | 200 | 1480 | 98% | 80% |
| 9 | Offretite | 6.4A | 250 | 4380 | 97% | 73% |
| 10 | HZSM—4 | 7.4A | 205 | 1550 | 92% | 73% |
| 11 | Beta | * | 250 | 4240 | 38% | 47% |
| 12 | Linde L | 7.1A | 195 | 1550 | 72% | 72% |
| 13 | HZSM—38 | * | 200 | 1585 | 94% | 73% |
| 14 | REY | 7.4A | 200 | 1620 | 89% | 85% |

NOTES:

\* Pore size not finally established, believed to be 6—7 Angstroms.

** Dealuminized, see Example 3.

TABLE II

Catalyst Comparison — Phenyldodecane Isomer Distribution

| Example | Catalyst | 2–∅ | 3–∅ | 4–∅ | 5–∅ | 6–∅ | % Linear |
|---|---|---|---|---|---|---|---|
| 7 | HZSM—12 | 92% | 8% | 0 | 0 | 0 | 78% |
| 8 | Mordenite (—A1) | 85% | 15% | 0 | 0 | 0 | 95% |
| 9 | Offretite | 79% | 14% | 5% | 1% | 1% | 75% |
| 10 | HZSM—4 | 57% | 25% | 8% | 5% | 5% | 90% |
| 11 | Beta | 57% | 18% | 10% | 7% | 8% | 53% |
| 12 | Linde L | 40% | 18% | 16% | 15% | 11% | 88% |
| 13 | HZSM—38 | 37% | 19% | 13% | 14% | 16% | 78% |
| 14 | REY | 25% | 20% | 18% | 19% | 18% | 92% |

0030088

The zeolites used in Examples 7—9, which can be used in the present process produce the 2-phenyldodecane isomer selectivity in very high yields with little or none of the other isomers produced as side-products. In contrast, the larger pore size zeolites of Examples 10—14 are seen to produce a relatively broader distribution of phenyldodecane isomers, making the 2-isomer difficult to isolate in significant amounts.

Both the oxidation of the 2-arylalkane to the corresponding hydroperoxide (Step 2) and the subsequent acid-catalyzed rearrangement of the resultant hydroperoxide (Step 3) are analogous to the well-known process for the manufacture of phenol from isopropylbenzene. The conventional technology used in that process is essentially the same as that utilized herein. See, for instance, the article entitled "Phenol" in the Kirk-Othmer ENCYCLOPEDIA OF CHEMICAL TECHNOLOGY, 2nd. edition, Vol. 15, p. 147 and INDUSTRIAL CHEMICALS, 4th. edition, p. 612, by Faith, Keyes & Clark.

The oxidation reaction may be conveniently carried out either in batch or continuous operation at from 75°C up to 130°C and at pressures ranging up to 1000 kPa. An appropriate base, preferably in aqueous solution, is used to maintain the pH of the reaction mixture at 7 to 9 to prevent decomposition of the hydroperoxide. It is also desirable to add a radical initiator to the reaction mix to optimize the conversion rate and selectivity to the desired hydroperoxide. Suitable radical initiators are well-known and the most preferable are the organic hydroperoxide, particularly the same aromatic hydroperoxide which is the desired product of the reaction. However, numerous other conventional free radical initiators may suitably be employed (e.g. metal oxide catalysts such as $MnO_2$). The source of oxygen for the formation of the hydroperoxide is normally an oxygen-containing gas (e.g. pure $O_2$ or air) which is brought into contact with the organic reactants by convenient means, such as continuously bubbling the gas through the reaction mixture under reaction conditions.

The hydroperoxide is cleaved and rearranged to the aromatic alcohol by bringing it into contact with an inorganic acid, such as $H_2SO_4$, and preferably at elevated temperature. Alternatively, the hydroperoxide, in suitable solvent, may be converted to the aromatic alcohol by means of a cation exchange resin.

Prior to carrying out the rearrangement, it is preferable that the hydroperoxide be separated from the crude reaction product mix, in order to maximize the efficiency of the cleavage reaction and also to recycle the unreacted starting materials to increase the yield and efficiency of the hydroperoxidation step. One suitable method of recovering the hydroperoxide is by crystallization from the crude product mix, but the preferred method comprises extraction with an aqueous base (e.g. NaOH) followed by treatment of the salt with $CO_2$ to regenerate the hydroperoxide.

Recycling of the unreacted starting materials, particularly after extraction of the hydroperoxide product, is preferred, especially in continuous operations. However, such recycling may result in an accumulation of essentially inert by-products which will act as diluents and thereby prove detrimental to the reaction. It is therefore of benefit to minimize the accumulation of undesirable by-products by withdrawing a portion of the recycle prior to returning it to the oxidation reactor. Another method of preventing or minimizing accumulation of by-products is to conduct the oxidation process in a cascade consisting of several reactors.

Oxidation and rearrangement of the 2-arylalkane produces the 2-alkanone and phenol. If the alkylating agent used in Step 1 was of a single molecular weight, then the 2-alkanone produced is likewise expected to be of a single molecular weight. When alkylating agents of varying molecular weight are employed, then a mixture of 2-alkanones results. Surprisingly, when alkylating agents of the same molecular weight but differing in the position of the expected reactive site (e.g. the position of the double bond in an olefin) are utilized in Step 1, a single 2-arylalkane isomer, and subsequently a single 2-alkanone, are the predominant reaction products in Steps 1 and 3, respectively. The phenol produced will have substantially the same aromatic structure, including original substituents, as the aromatic compound used in Step 1, except with the addition thereto of the phenolic hydroxyl group.

## Claims

1. A method for co-producing 2-alkanones of at least five carbon atoms and aromatic alcohols, the method comprising:
(A) producing a 2-arylalkane, in which the alkyl moiety has at least five carbon atoms by reacting an aromatic compound with an alkylating agent having one or more available reactive alkyl groups of at least five carbon atoms in the linear hydrocarbon chain in the presence of a selective crystalline zeolite catalyst having a crystal structure having channels or networks of pores with openings having a major dimension from 6 to 7 Angstroms (0.6 to 0.7 nm), the reaction being carried out at a temperature from 50°C to 500°C and a pressure from 25 to 25000 kPa;
(B) oxidizing the 2-arylalkane with an oxidizing agent to produce the hydroperoxide thereof; and
(C) cleaving and rearranging the hydroperoxide by contacting said hydroperoxide with an inorganic acid or a cation exchange resin to yield a 2-alkanone of substantially the same carbon chain length as the alkyl moiety and a phenol.

2. A method as defined in Claim 1 in which the alkyl moiety has from six to twenty carbon atoms in the linear hydrocarbon chain.

3. A method as defined in Claim 1 or 2 in which the aromatic compound is benzene.

4. A method as defined in Claim 1 or 2 in which the aromatic compound comprises a benzene ring having from one to two substituents thereon.

5. A method as defined in any of Claims 1 to 4 in which the aromatic compound is reacted with the alkylating agent at a temperature from 100°C to 350°C and a pressure from 100 to 5000 kPa.

6. A method as defined in any of Claims 1 to 5 in which the selective zeolite is cancrinite, gmelinite, or a synthetic or naturally occurring isotype thereof.

7. A method as defined in any of Claims 1 to 5 in which the selective zeolite has the crystal structure of mordenite.

8. A method as defined in any of claims 1 to 5 in which the selective zeolite is ZSM—12.

9. A method as defined in any of Claims 1 to 5 in which the selective zeolite has the crystal structure of offretite.

10. A method as defined in any of Claims 1 to 8 in which the zeolite is steamed prior to use.

## Patentansprüche

1. Verfahren zur gleichzeitigen Herstellung von 2-Alkanonen mit mindestens fünf Kohlenstoffatomen und von aromatischen Alkoholen, bei dem
(A) ein 2-Arylalkan· mit einer mindestens fünf Kohlenstoffatome aufweisenden Alkylgruppe hergestellt wird, in dem eine aromatische Verbindung mit einem Alkylierungsmittel, das eine oder mehrere zugängliche reaktive Alkylgruppen mit mindestens fünf Kohlenstoffatomen in der geraden Kohlenwasserstoffkette aufweist, in Gegenwart eines selektiven kristallinen Zeolitkatalysators, der eine Kristallstruktur mit Kanälen oder einem Netz von Poren aufweist, deren Öffnungen eine maximale **Abmessung zwischen 6 und 8 Å** (0,6—0,7 nm) besitzen, umgestetzt wird, wobei die Umsetzung bei einer Temperatur zwischen 50°C und 500°C und bei einem **Druck zwischen 25 und 25 000 kPa erfolgt;**
(B) das 2-Arylalkan mit einem Oxidationsmittel oxidiert wird, um das Hydroperoxid zu bilden; und
(C) das Hydroperoxid gespalten und umgelagert wird, indem das Hydroperoxid mit einer anorganischen Säure oder einem Kationenaustauschharz in Berührung gebracht wird, um ein 2-Alkanon mit im wesentlichen der gleichen Kohlenstoffkettenlänge wie die Alkylgruppe und ein Phenol zu bilden.

2. Verfahren nach Anspruch 1, bei dem die Alkylgruppe 6 bis 20 Kohlenstoffatome in der linearen Kohlenwasserstoffkette aufweist.

3. Verfahren nach Anspruch 1 oder 2, wobei die aromatische Verbindung Benzol ist.

4. Verfahren nach Anspruch 1 oder 2, wobei die aromatische Verbindung einen Benzolring mit einem oder zwei Substituenten umfaßt.

5. Verfahren nach einem der Ansprüche 1 bis 4, wobei die aromatische Verbindung mit dem Alkylierungsmittel bei einer Temperatur zwischen 100°C und 350°C bei einem Druck von 100 bis 5000 kPa umgesetzt wird.

6. Verfahren nach einem der Ansprüche 1 bis 5, wobei der selektive Zeolit Kancinarit, Gmelinit oder ein synthetischer oder natürlich vorkommender Isotyp davon ist.

7. Verfahren nach einem der Ansprüche 1 bis 5, wobei der selektive Zeolit die Kristallstruktur von Mordenit aufweist.

8. Verfahren nach einem der Ansprüche 1 bis 5, wobei der selektive Zeolit ZSM—12 ist.

9. Verfahren nach einem der Ansprüche 1 bis 5, wobei der selektive Zeolit die Kristallstruktur des Offretit aufweist.

10. Verfahren nach einem der Ansprüche 1 bis 8, wobei der Zeolit vor dem Einsatz Wasserdampf ausgesetzt wird.

## Revendications

1. Procédé pour la coproduction d'alcanones-2 d'au moins 5 atomes de carbone et d'alcools aromatiques, ce procédé comprenant:
(A) la production d'un aryl-2 alcane dont le fragment alkyle a au moins 5 atomes de carbone par réaction d'un composé aromatique avec un agent d'alkylation ayant un ou plusieurs radicaux alkyles d'au moins 5 atomes de carbone disponibles dans la chaîne hydrocarbonée linéaire, en présence d'un catalyseur constitué d'une zéolite cristalline sélective ayant une structure cristalline ayant des canaux ou des réseaux de pores avec des ouvertures ayant une dimension principale de 0,6 à 0,7 nm, la réaction étant effectuée à une température de 50°C à 500°C et sous une pression de 25 à 25 000 kPa;
(B) l'oxydation de l'aryl-2 alcane avec un agent oxydant pour produire son hydroperoxyde; et
(C) le clivage et le réarrangement de l'hydroperoxyde par contact dudit hydroperoxyde avec un acide minéral ou une résine échangeuse de cations pour produire une alcanone-2 ayant pratiquement la même longeur de la chaîne carbonée que le fragment alkyl et un phénol.

2. Procédé selon la revendication 1 dans lequel le fragment alkyle a 6 à 20 atomes de carbone dans la chaîne hydrocarbonée linéaire.

3. Procédé selon l'une des revendications 1 ou 2 dans lequel le composé aromatique est le benzène.

4. Procédé selon l'une des revendications 1 ou 2 dans lequel le composé aromatique comprend un cycle benzène portant un ou deux substituants.

5. Procédé selon l'une quelconque des revendications 1 à 4 dans lequel le composé aromatique réagit avec l'agent d'alkylation à une température de 100°C à 350°C sous une pression de 100 à 5 000 kPa.

6. Procédé selon l'une quelconque des revendications 1 à 5 dans lequel la zéolite sélective est la cancrinite, la gmelinite ou un de leurs isotypes synthétiques ou naturels.

7. Procédé selon l'une quelconque des revendications 1 à 5 dans lequel la zéolite sélective a la structure cristalline de la mordénite.

8. Procédé selon l'une quelconque des revendications 1 à 5 dans lequel la zéolite sélective est la zéolite ZSM—12.

9. Procédé selon l'une quelconque des revendications 1 à 5 dans lequel la zéolite sélective a la structure cristalline de l'offretite.

10. Procédé selon l'une quelconque des revendications 1 à 8 dans lequel la zéolite est traitée par la vapeur d'eau avant l'emploi.